# EUROPEAN PATENT APPLICATION

(11) **EP 4 745 122 A1**
(43) Date of publication of application: **20.05.2026**
(21) Application number: 24838879.5
(22) Date of filing: 12.07.2024
(51) Int. Cl.: C07D 207/34

(54) **METHOD FOR PREPARING PYRROLE CARBOXYLATE COMPOUND**

(30) Priority: 12.07.2023 CN 202310858213
(71) Applicant: Sunshine Lake Pharma Co., Ltd., Dongguan, Guangdong 523000 (CN)
(72) Inventor: WANG, Jiancheng, Dongguan, Guangdong 523871 (CN); ZHANG, Yingxun, Dongguan, Guangdong 523871 (CN); YANG, Chuanwen, Dongguan, Guangdong 523871 (CN); CHEN, Hongfen, Dongguan, Guangdong 523871 (CN); ZUO, Yinglin, Dongguan, Guangdong 523871 (CN)
(74) Representative: Hoffmann Eitle
(86) International application number: PCT/CN2024/105063
(87) International publication number: WO 2025/011627

(57) **Abstract**

This invention discloses a method for preparing a pyrrole carboxylic acid compound. Wherein the pyrrole carboxylic acid compound described in this invention can be used to prepare a pyrrole amide compound that serve as a mineralocorticoid receptor antagonist. Specifically, the provided preparation method has mild preparation conditions, is simple in operation, is safe and controllable, has high yield, and is suitable for industrial production.

## Description

### FIELD OF THE INVENTION

This invention relates to the field of medicinal chemistry, specifically to methods for preparing a pyrrole carboxylic acid compound, and further to a method for preparing a pyrrole amide compound using the pyrrole carboxylic acid compound as an important intermediate. The pyrrole carboxylic acid compound described in this invention can be used to prepare a pyrrole amide compound that serve as a mineralocorticoid receptor antagonist.

### BACKGROUND ART

International patent application WO2021078135A1 discloses a class of pyrrole amide compounds and uses thereof. These compounds can be used as mineralocorticoid receptor antagonists and have potential therapeutic effects on diseases such as hyperaldosteronism, hypertension, chronic heart failure, sequelae of myocardial infarction, cirrhosis, non-alcoholic steatohepatitis, chronic kidney disease, diabetic nephropathy, renal failure, fibrosis and/or stroke. Specifically, the international application discloses a compound as shown in formula (A) (i.e., compound (*S*)-*N*-(3-fluoro-4-(methylsulfonyl)phenyl)-1-(2-hydroxyethyl)-4-methyl-5-(2-(trifluoromethyl)phenyl)-1*H*-pyrrol e-3-carboxamide), and also discloses the following preparation method: using ethyl 4-methyl-5-(2-(trifluoromethyl)phenyl)-1*H*-pyrrole-3-carboxylate as a raw material, a racemic compound of formula (A) is obtained through substitution, hydrolysis, acyl chlorination, condensation, and deprotection reactions, and then the compound of formula (A) is obtained by chiral column resolution. The method uses a chiral resolution method to separate stereoisomers. This separation method has high requirements for instruments and low yield. Overall, this preparation method has high production costs, poor atom economy, significant environmental pollution, and harsh reaction conditions, making it unsuitable for industrial-scale production.

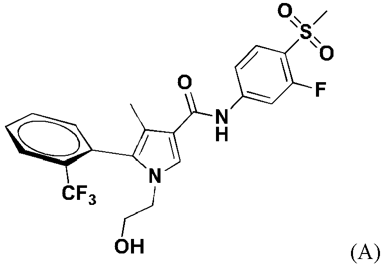

International patent application WO2022228215A1 discloses a method for preparing the compound of formula (A). In this method, lipase is used to chirally resolve the racemic raw material methyl 1-(2-hydroxyethyl)-4-methyl-5-(2-(trifluoromethyl)phenyl)-1*H*-pyrrole-3-carboxylate to obtain the intermediate (*S*)-1-(2-hydroxyethyl)-4-methyl-5-(2-(trifluoromethyl)phenyl)-1*H*-pyrrole-3-carboxylate, followed by benzyl protection, ester hydrolysis, and ammoniation to obtain the amide intermediate (*S*)-1-(2-(benzyloxy)ethyl)-4-methyl-5-(2-(trifluoromethyl)phenyl)-1*H*-pyrrole-3-carboxamide, and which finally undergoes condensation and deprotection to obtain the compound of formula (A). This method obtains the intermediate by enzymatic resolution, avoiding the use of chiral column resolution. However, the total yield of the reaction to prepare the amide intermediate from the racemic raw material is not high, and the total yield of the compound of formula (A) is also not high. A method for preparing the compound of formula (A) with higher yield and lower cost is needed.

### SUMMARY

This invention provides intermediates that can be used to prepare the compound of formula (A) and a preparation method thereof; the intermediates include the compounds represented by the general formulae and/or structural formulae described in this invention.

This invention relates to a method for preparing pyrrole carboxylic acid compound, and also relates to the important intermediate used in the method and a preparation method thereof. The preparation method described in this invention is characterized by mild conditions, simple operation, safety and controllability, high yield, and is suitable for industrial production.

The pyrrole carboxylic acid compound prepared by the method described in this invention can be used to prepare the compound of formula (A).

In one aspect, the present invention provides a compound of formula (I), 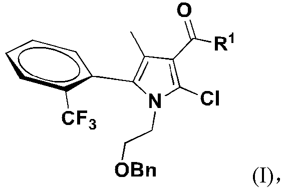 wherein, R¹ is OH, NH₂, C₂₋₄ alkoxy or benzyloxy, each of the C₂₋₄ alkoxy and benzyloxy is optionally substituted with 1, 2, 3 or 4 substituents independently selected from halogen, C₁₋₄ alkyl, C₁₋₄ haloalkyl, C₁₋₄ alkoxy and C₁₋₄ haloalkoxy.

In some embodiments, the R¹ is OH, NH₂, C₂₋₄ alkoxy (such as ethoxy, *n*-propoxy, isopropoxy, *n*-butoxy or *tert*-butoxy, etc.) or benzyloxy.

In other aspect, the present invention provides a compound having one of the following structures:

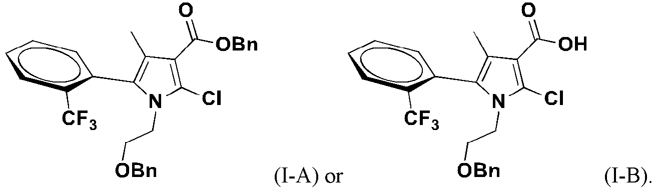

Optionally, the present invention relates to the use of the compound represented by formula (I-A) or (I-B) in the preparation of the compound represented by formula (II). Optionally, the method for preparing the compound represented by formula (II) is as described in the present invention. Optionally, the compound represented by formula (I-A) or (I-B) of the present invention can be used as an intermediate in the method for preparing the compound represented by formula (II) of the present invention.

In one aspect, the present invention provides a method for preparing the compound of formula (II), 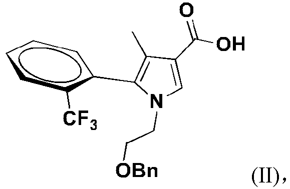 wherein, the method comprises:
Step c) The compound of formula (I-B) is dechlorinated in a solvent under suitable conditions to give the compound of formula (II),

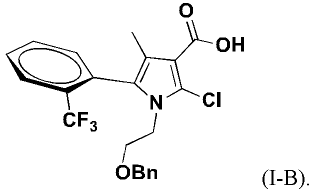

In some embodiments, the reaction solvent in step c) is methanol, ethanol, isopropanol, *tert*-butanol, or a mixed solvent composed of any of them (*i.e.,* methanol, ethanol, isopropanol or *tert*-butanol) with other solvent in any combination (*e.g.*, in any proportion). In other embodiments, the other solvent includes, but is not limited to, tetrahydrofuran, DMF, ethyl acetate, methyl *tert*-butyl ether, ethylene glycol dimethyl ether, ethylene glycol monomethyl ether, toluene, water, or any combination thereof. In some embodiments, the reaction solvent in step c) is methanol, ethanol, ethanol/toluene, ethanol/water, ethanol/toluene/water, ethanol/tetrahydrofuran, or ethanol/tetrahydrofuran/water. In some embodiments, the reaction solvent in step c) is ethanol, ethanol/water or ethanol/toluene/water. Optionally, the ethanol/water in this invention refers to a mixed solvent composed of ethanol and water, and the ethanol/toluene/water refers to a mixed solvent composed of ethanol, toluene and water. Unless otherwise specified, the solvents in the mixed solvent may be mixed in any proportion.

In some embodiments, the reaction solvent in step c) of the present invention is ethanol/toluene/water, wherein the volume ratio of ethanol to water is from 4:1 to 20:1, preferably from 4:1 to 18:1, more preferably from 4:1 to 17:1; even more preferably, the volume ratio of ethanol to water is from 5:1 to 16:1. In other embodiments, the reaction solvent in step c) of the present invention is ethanol/toluene/water, wherein the volume ratio of ethanol to water is from 8:1 to 12:1, or from 9:1 to 11:1. In other embodiments, the reaction solvent in step c) of the present invention is ethanol/toluene/water, wherein the volume ratio of ethanol to water is 5:1, 6:1, 8:1,9:1,10:1 or 12:1.

In some embodiments, the reaction solvent in step c) of the present invention is ethanol/toluene/water, wherein the volume ratio of toluene to water is from 1:1 to 15:1, preferably from 1:1 to 12:1, more preferably from 1:1 to 11:1; even more preferably, the volume ratio of toluene to water is from 1:1 to 10:1. In other embodiments, the reaction solvent in step c) of the present invention is ethanol/toluene/water, wherein the volume ratio of toluene to water is from 3:1 to 7:1, or from 4:1 to 6:1. In other embodiments, the reaction solvent in step c) of the present invention is ethanol/toluene/water, wherein the volume ratio of toluene to water is 1:1, 2:1, 4:1, 5:1, 6:1 or 10:1.

In some embodiments, the reaction solvent in step c) of the present invention is ethanol/toluene/water, wherein the volume ratio of ethanol to water is from 4:1 to 20:1, and the volume ratio of toluene to water is from 1:1 to 15:1; preferably, the volume ratio of ethanol to water is from 4:1 to 17:1, and the volume ratio of toluene to water is from 1:1 to 11:1; or preferably, the volume ratio of ethanol to water is from 5:1 to 16:1, and the volume ratio of toluene to water is from 1:1 to 10:1; more preferably, the volume ratio of ethanol to water is from 6:1 to 10:1, and the volume ratio of toluene to water is from 1:1 to 5:1.

In other embodiments, the reaction solvent in step c) of the present invention is ethanol/toluene/water, wherein the volume ratio of ethanol, toluene and water is 10:5:1, 10:2:1, 6:4:1, 8:2:1 or 9:1:1.

In some embodiments, the reaction in step c) of the present invention is carried out in the presence of a transition metal catalyst. Optionally, the transition metal catalyst is palladium-carbon, other palladium-containing catalysts (such as palladium acetate, etc.), nickel or nickel-containing catalysts (such as nickel chloride), etc. In other embodiments, the reaction in step c) of the present invention is carried out in the presence of a transition metal catalyst, wherein the transition metal catalyst is a palladium-carbon catalyst, a palladium acetate catalyst or a nickel catalyst.

In some embodiments, the reaction in step c) of the present invention is carried out in the presence of a palladium-carbon catalyst. Optionally, the amount of the palladium-carbon catalyst is from 1% to 20% of the mass of the compound of formula (I-B); in some embodiments, the amount of the palladium-carbon catalyst is from 2% to 15% or from 2% to 11% of the mass of the compound of formula (I-B); in other embodiments, the amount of the palladium-carbon catalyst is from 3% to 10% of the mass of the compound of formula (I-B).

In some embodiments, the reaction in step c) of the present invention is carried out in the presence of ammonium formate, sodium formate, sodium hydrogen phosphate (such as disodium hydrogen phosphate, etc.), acid, triethylamine or hydrogen.

In some embodiments, the reaction in step c) of the present invention is carried out in the presence of ammonium formate, sodium formate or hydrogen. In some other embodiments, the reaction in step c) of the present invention is carried out in the presence of ammonium formate or sodium formate, wherein the amount of ammonium formate or sodium formate is from 1 equivalent to 5 equivalents, preferably from 1 equivalent to 3 equivalents, more preferably from 1 equivalent to 2 equivalents, and even more preferably from 1 equivalent to 1.5 equivalents. In some other embodiments, the reaction in step c) of the present invention is carried out in the presence of sodium formate, wherein the amount of sodium formate is from 1 equivalent to 2 equivalents, preferably from 1 equivalent to 1.5 equivalents. In some embodiments, the reaction in step c) of the present invention is carried out in the presence of sodium formate, wherein the amount of sodium formate is 1.5 equivalents. Optionally, the sodium formate described in this invention may be sodium formate dihydrate or anhydrous sodium formate. Unless otherwise specified, the equivalent referred to herein means the ratio of the molar amount of the reagent sodium formate to the molar amount of the reaction substrate compound of formula (I-B); for example, the term "the amount of sodium formate is 1.5 equivalents" means that the ratio of the molar amount of sodium formate to the molar amount of the compound of formula (I-B) is 1.5.

In some embodiments, the reaction in step c) of the present invention is carried out under heating conditions. Optionally, the heating conditions are heating to 45 °C-75 °C; in some embodiments, the heating conditions are heating to 55 °C-70 °C; in some embodiments, the heating conditions are heating to 60 °C-65 °C.

In some embodiments, the method for preparing the compound of formula (II) of the present invention further includes a post-treatment method for the reaction in step c). In other embodiments, the post-treatment method in step c) of the present invention includes, but is not limited to: after the reaction is completed, the reaction solution is filtered, washed, concentrated to remove the solvents (such as ethanol and toluene), the residue is dissolved in ethanol, the pH is adjusted with hydrochloric acid, the resulting solution is added dropwise to ice water, stirred at room temperature, filtered and optionally washed, the filter cake is collected and dried to obtain the target compound. Optionally, the filtration may be suction filtration or filtration through celite pad. Optionally, the first wash is performed using the reaction solvent, such as ethanol; the second wash may optionally be performed, for example, with water. Optionally, other solvent, such as DMAC (i.e., N,N-dimethylacetamide), may be added prior to the concentration. Optionally, the drying may be vacuum drying or heated vacuum drying (e.g., vacuum drying at 45 °C-60 °C). Optionally, after the reaction in step c) is completed, other suitable post-treatment methods may be used.

The method described in this invention can effectively control the generation of reaction impurities and obtain the intermediate compound shown in formula (II) with high purity and high yield.

In some embodiments, the method for preparing the compound of formula (II) of the present invention further includes a method for preparing the compound of formula (I-B), wherein, the method for preparing the compound of formula (I-B) comprises:
Step a) The compound of formula (I-1) reacts with a benzyl protecting group reagent to obtain the compound of formula (I-A); 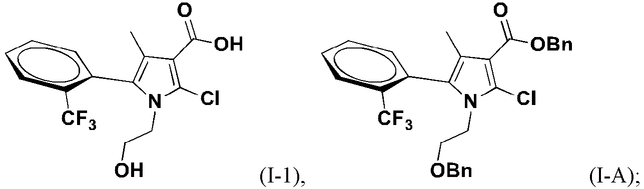
Step b) The compound of formula (I-A) is debenzylated under suitable conditions to obtain the compound of formula (I-B);

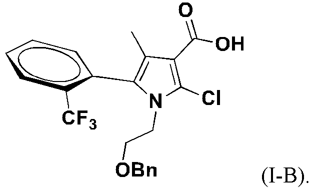

In some embodiments, the benzyl protecting group reagent in step a) is a halobenzyl group, optionally benzyl bromide.

In some embodiments, step a) is carried out in the presence of sodium tert-amylate.

In some embodiments, the reaction solvent in step a) is *N,N*-dimethylacetamide.

In some implementations, the reaction temperature in step a) is 25 °C to 60 °C. In some implementations, the reaction temperature is 35 °C to 55 °C. In some implementations, the reaction temperature is 35 °C to 45 °C. In some implementations, the reaction temperature is 45 °C to 55 °C.

In some embodiments, the reaction in step b) is carried out in the presence of a base, and the base is sodium carbonate, potassium carbonate, cesium carbonate, potassium phosphate, lithium hydroxide, potassium hydroxide or sodium hydroxide.

In some embodiments, the reaction in step b) is carried out in a solvent, and the solvent is acetone, acetonitrile, *N,N*-dimethylformamide, *N,N*-dimethylacetamide, DMSO, methanol, ethanol, THF, methyl *tert*-butyl ether, water or any combination thereof.

In some embodiments, the reaction in step b) is carried out under heating conditions, and the heating conditions refer to heating to 60 °C to 80 °C; in other embodiments, the heating conditions refer to heating to 65 °C to 75 °C.

In other aspect, the present invention provides the use of a compound having one of the following structures in the preparation of a pyrrole amide compound used as a mineralocorticoid receptor antagonist.

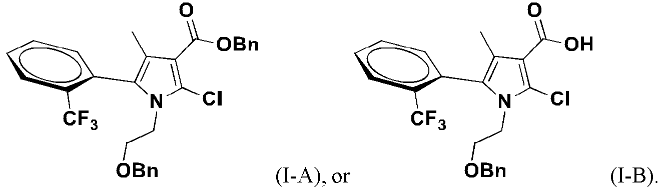

In some embodiments, the pyrrole amide compound used as a mineralocorticoid receptor antagonist according to the present invention is (*S*)-*N*-(3-fluoro-4-(methanesulfonyl)phenyl)-1-(2-hydroxyethyl)-4 -methyl-5-(2-(trifluoromethyl)phenyl)-1*H*-pyrro le-3-carboxamide (i.e., the compound of formula (A)).

In some embodiments, the use of the compound of formula (II) of the present invention in the preparation of the compound of formula (A), wherein the compound of formula (II) is converted into the compound of formula (A) (i.e., (*S*)-*N*-(3-fluoro-4-(methylsulfonyl)phenyl)-1-(2-hydroxyethyl)-4-methyl-5-(2-(trifluoromethyl)phenyl)-1*H*-pyrrol e-3-carboxamide) via the following method:

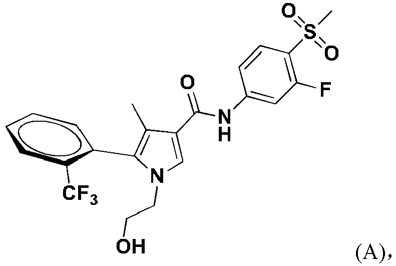

The compound of formula (II) is aminated under suitable conditions to give the compound of formula (III), and then reacts with the compound of formula (IV) to give the compound of formula (V). The compound of formula (V) is deprotected to remove the benzyl group to give the target compound; 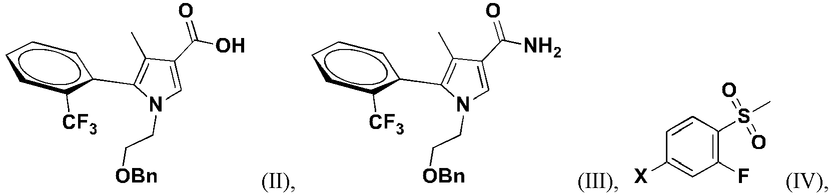 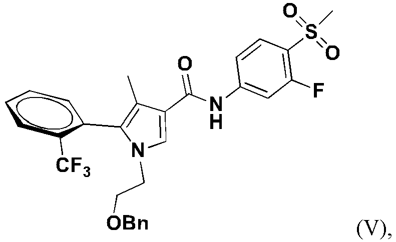 wherein, X is Br or I.

Optionally, the compound of formula (II) of the present invention can be prepared according to the method described in this invention.

Optionally, the compound of formula (A) can be prepared using the compound of formula (II) of the present invention by referring to the methods disclosed in the prior art such as WO2022228215A1.

In one aspect, the method for preparing the compound of formula (II) or the method for preparing the compound of formula (I-B) according to the present invention further includes the method for preparing the compound of formula (I-1). 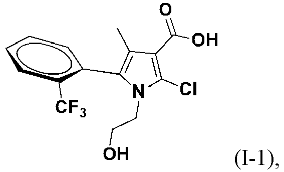 wherein, the method comprises:
1a) The compound of formula (I-0) reacts with an optically active amine to obtain the corresponding salt of the optically active amine of the compound of formula (I-1), and
1b) The salt obtained in step 1a) reacts to give the compound of formula (I-1);

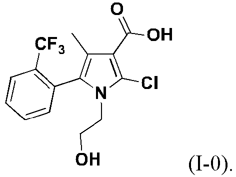

In some embodiments, the optically active amine of the present invention is an optically active amine havig a quinine skeleton.

In some embodiments, the optically active amine having a quinine skeleton of the present invention is quinine, hydroquinine, quinidine, cinchonine or cinchonidine.

In some embodiments, the salt of the optically active amine of the compound described in Formula (I-1) of the present invention is the salt formed by the compound shown in Formula (I-1) and the optically active amine. In other embodiments, the salt of the optically active amines of the compound of Formula (I-1) of the present invention is a quinine salt of the compound of Formula (I-1), a hydroquinine salt of the compound of Formula (I-1), a quinidine salt of the compound of Formula (I-1), a cinchonine salt of the compound of Formula (I-1), or a cinchonidine salt of the compound of Formula (I-1).

In some embodiments, the reaction in step 1b) of the present invention is a hydrolysis reaction.

In some embodiments, the reaction in step 1b) of the present invention is carried out under acidic conditions.

In some embodiments, the acidic conditions of the present invention are the conditions for the presence of hydrochloric acid, sulfuric acid, hydrobromic acid or citric acid.

In some embodiments, the reaction solvent in step 1b) of the present invention is *N,N*-dimethylformamide (DMF), *N,N*-dimethylacetamide (DMAC), acetonitrile, tetrahydrofuran (THF), ethanol, acetone, isopropyl acetate, ethyl acetate, methyl tert-butyl ether, water, dimethoxyethane or any combination thereof.

In some embodiments, the reaction solvent in step 1a) of the present invention is *N,N*-dimethylformamide (DMF), *N,N*-dimethylacetamide (DMAC), acetonitrile, tetrahydrofuran (THF), ethanol, acetone, isopropyl acetate, ethyl acetate, methyl *tert*-butyl ether, water, dimethoxyethane or any combination thereof.

In some embodiments, the reaction temperature in step 1a) of the present invention is room temperature to 100 °C; in some embodiments, the reaction temperature in step 1a) of the present invention is 50 °C-80 °C; in some embodiments, the reaction temperature in step 1a) of the present invention is 55 °C-65 °C.

In some embodiments, the preparation method of the compound of Formula (I-1) of the present invention further comprises: spinning the mixed solution obtained after removing the salt of the optically active amine of the compound of Formula (I-1) in step 1a), adding a suitable solvent, isomerizing by heating to obtain a racemic compound of Formula (I-0), then reacting with an optically active amine to obtain a salt of the optically active amine of the compound of Formula (I-1). In general, the optically active amine described here is the same as those used in step 1a). Optionally, the suitable solvent includes but is not limited to, DMF, etc.

In other embodiments, optionally, the isomerization refers to the racemization of the atropisomer in a solution by a mean such as heating to obtain racemic compound (i.e., the compound of Formula (I-0)). Optionally, a salt of the optically active amine of the compound of Formula (I-1) may be prepared by the racemic compound through the method of step 1a) of the present invention. Optionally, the compound of Formula (I-1) can be prepared by the salt of the optically active amine of the compound of Formula (I-1) through the method of step 1b) of the present invention.

The preparation method of the compound of Formula (I-1) shown in the present invention is simple to operate, has high yield and high ee/de value of the obtained product, and the by-product of R-configuration obtained by this method can be recycled through the racemization described in the present invention, thereby further improving the yield of a compound of Formula (I-1) (up to more than 60%).

In other aspect, the present invention provides a salt formed by a compound shown in Formula (I-1) and an optically active amine,

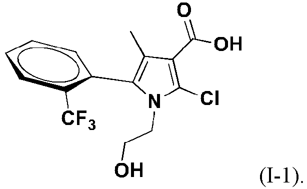

In some embodiments, the optically active amine of the present invention is quinine, hydroquinine, quinidine, cinchonine or cinchonidine. Optionally, the optically active amines have the following structures, respectively.

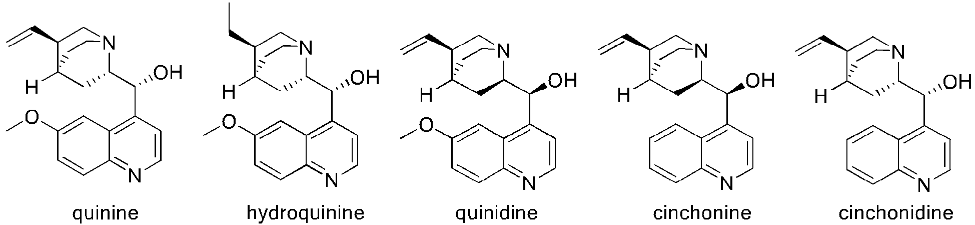

Preferably, the salt formed by the compound of Formula (I-1) of the present invention and the optically active amine is a quinine salt of the compound of Formula (I-1) compound, a hydrogenated quinine salt of the compound of Formula (I-1), a quinidine salt of the compound of Formula (I-1), a cinchonine salt of the compound of Formula (I-1), or a cinchonidine salt of the compound of Formula (I-1).

The compounds of Formulae (I-0), (I-1), (I-A), (I-B), (II) and/or (III) of the present invention are used to prepare the compound of Formula (A) of the present invention. Preferably, the method for preparing the compound of Formula (A) using the aforementioned compounds is as described in the present invention.

### DEFINITIONS AND GENERAL TERMINOLOGY

In the present invention, "room temperature" refers to a temperature from about 10°C to about 40°C. In some embodiments, "room temperature" refers to a temperature from about 20 °C to about 30 °C; in other embodiments, "room temperature" refers to 20°C, 22.5°C, 25°C, 27.5°C and the like.

In the context of the present invention, all values disclosed herein are approximate. The value of each number may vary by 1%, 2%, 5%, 7%, 8% or 10%. When a number with an N value is made public, any number within N +/- 1%, N +/- 2%, N +/- 3%, N +/- 5%, N +/- 7%, N +/- 8%, or N +/- 10% will be disclosed clearly, wherein "+/-" means plus or minus. Whenever a lower limit, DL, and an upper limit, DU, of a numerical range are disclosed, any values falling within the disclosed range are explicitly disclosed.

After all the reaction steps according to the present invention proceeds to a certain extent , such as the raw materials are consumed about more than 70%, more than 80%, more than 90%, or more than 95%, or completely through detection, the post-treatment, such as cooling, collecting, extracting, filtering, separating, purifying or a combination thereof, is performed. The reaction can be monitored by conventional method such as thin-layer chromatography (TLC), high performance liquid chromatography (HPLC), gas chromatography (GC), and the like. The reaction solution can be post-treated by conventional methods; for example, the crude product can be collected by removing the reaction solvent through vacuum evaporation or conventional distillation and is used directly in the next reaction; or the crude product can be obtained by filtration and is used directly in the next reaction; or the crude product can be get by pouring the supernatant liquid after standing and is used directly in the next operation; or appropriate organic solvents or their combinations are selected for extraction, distillation, crystallization, column chromatography, rinsing, pulping and other purification steps.

The term "approximately" or "about" in the present invention is used to modify a numerical value with a difference of 10% between the top and the bottom. The term "approximately" or "about" in the present invention is used to modify a numerical value with a difference of 5% between the top and the bottom. The term "approximately" or "about" in the present invention is used to modify a numerical value with a difference of 3% or 2% or 1% between the top and the bottom. It is understandable that the numerical margin of error of an "approximately" or "about" modification is an actual or reasonable margin of error depending on the value it modifies.

The term "optional" or "optionally" refers to that a subsequently described event or circumstance may but need not occur, i.e., the description includes instances where the event or circumstance occurs and instances in which it does not.

In each step of the reaction process described in the present invention, the reaction raw material or other reagent can be added to the reaction system by dropping. The dropping process and each step reaction are carried out under certain temperature conditions, and any temperature suitable for being used in each dropping process or each reaction process is included in the present invention. Additionally, many similar modifications in the art, equivalent replacements, or temperature and temperature scope which are equivalent to those described in the invention, all are deemed to be included in the present invention. The invention gives the optimal temperature or temperature range of each dropping process, and the optimal reaction temperature or reaction temperature range of each reaction.

The expressions "solvent 1", "solvent 2", "solvent a", "solvent b", "baes a", "base b" and the like described in the present invention use Arabic numerals 1, 2, 3...... after "solvent" or "base" or the letters a, b, c...... , just to better distinguish the solvents or bases used in the individual steps, the Arabic numerals or letters used have no special meaning. For example, solvent 1 includes any solvent suitable for the reaction of preparing the compound of Formula (II) by reacting the compound having Formula (III) with a compound shown in Formula (IV), including but not limited to toluene, dioxane, dimethyl sulfoxide, tert-butanol, tert-amyl alcohol, dimethyl ether (DME), cyclopentyl methyl ether (CPME), N,N-dimethylacetamide, water or any combination thereof.

The solvent used for the reaction of the invention is not particularly restricted, any solvent is contained in the invention so long as it can dissolve the raw materials to a certain extent and doesn't inhibit the reaction. Additionally, many similar modifications in the art, equivalent replacements, or solvent, solvent composition and the solvent composition with different proportions which are equivalent to those described in the invention, all are deemed to be included in the present invention. The present invention gives the preferred solvent for each reaction step.

The product of each reaction step of the present invention can be purified by recrystallization under suitable conditions. The solvent used for the recrystallization is not particularly restricted, any solvent is contained in the invention so long as it can dissolve the crude product and the recrystallized product can precipitate out under certain conditions. Additionally, many similar modifications in the art, equivalent replacements, or solvent, solvent composition and the solvent composition with different proportions which are equivalent to those described in the invention, all are deemed to be included in the present invention. Wherein the solvent could be alcohol, ethers, alkanes, halohydrocarbons, esters, ketones, aromatic hydrocarbons, acetonitrile, acetic acid, water, DMF, or a combination thereof. Such as water, acetic acid, methanol, ethanol, *n*-propanol, *i*-propanol, *n*-butanol, *i*-butanol, *tert*-butanol, petroleum ether, *n*-pentane, *n*-hexane, *n*-heptane, cyclohexane, DMF, N, *N*-dimethylacetamide, tetrahydrofuran, ethyl ether, isopropyl ether, dioxane, methyl tertiary butyl ether, dimethoxylethane, diethylene glycol dimethyl ether, triethylene glycol dimethyl ether, dichloromethane, 1,2-dichloroethane, chloroform, tetrachloromethane, ethyl acetate, isopropyl acetate, acetone, butanone, benzene, toluene, xylene or a combination thereof.

The amount of water in the solvent described in the present invention is not particularly restricted, that is, the content of water in the solvent does not affect the occurrence of the reaction described in the present invention. So long as the solvent containing a certain amount of water can be used in the reaction disclosed herein, which is deemed to be included in the present invention. The amount of water in the solvent is approximately less than 0.05%, less than 0.1%, less than 0.2%, less than 0.5%, less than 5%, less than 10%, less than 25%, less than 30%, or 0%. In some embodiments, the amount of water in the solvent is within a certain range, which is more conducive to the progress of the reaction; for example, in the step where ethanol is used as the reaction solvent, absolute ethanol is used to facilitate the reaction. In some embodiments, the amount of water in the solvent is outside a certain range, which may affect the progress of the reaction (e.g., affect the yield of the reaction), but does not affect the occurrence of the reaction.

### GENERAL SYNTHETIC PROCEDURES

In the present invention, if the chemical name of the compound doesn't match the corresponding structure, the compound is characterized by the corresponding structure.

In the examples described below, unless otherwise indicated all temperatures are set forth in degrees (°C) Celsius. Unless otherwise indicated, all kinds of materials and reagents are purchased from commodity suppliers and are not further purified when used, and some materials can be prepared according to well-known methods in the art.

1H NMR spectra were recorded by a Bruker Avance 400 MHz spectrometer or Bruker Avance III HD 600 spectrometer, using CDCl₃, *d*₆-DMSO, CD₃OD, D₂O or *d*₆-acetone (reported in ppm) as solvent, and using TMS (0 ppm) or chloroform (7.25 ppm) as the reference standard. When peak multiplicities are reported, the following abbreviations are used: s (singlet), d (doublet), t (triplet), m (multiplet), br (broadened), dd (doublet of doublets), dt (doublet of triplets), td (triplet of doublets), ddd (doublet of doublet of doublets), ddt (doublet of doublet of triplets), dddd (doublet of doublet of doublet of doublets). Coupling constants, when given, were reported in Hertz (Hz).

Low-resolution mass spectral (MS) data were also determined on an Agilent 6320 series LC-MS spectrometer equipped with G1312A binary pumps and a G1316A TCC (Temperature Control of Column, maintained at 30 °C), a G1329A autosampler and a G1315B DAD detector were used in the analysis. An ESI source was used on the LC-MS spectrometer.

Low-resolution mass spectral (MS) data were also determined on an Agilent 6120 series LC-MS spectrometer equipped with G1311A binary pumps, a G1316A TCC (Temperature Control of Column, maintained at 30 °C), a G1329A autosampler and a G1315D DAD detector were used in the analysis. An ESI source was used on the LC-MS spectrometer.

Both LC-MS spectrometers were equipped with an Agilent Zorbax SB-C18, 2.1 x 30 mm, 5 µm column. Injection volume was decided by the sample concentration. The flow rate was 0.6 mL/min. The HPLC peaks were recorded by UV-Vis wavelength at 210 nm and 254 nm. The mobile phase was 0.1% formic acid in acetonitrile (phase A) and 0.1% formic acid in ultrapure water (phase B). The gradient elution conditions were showed in Table 1:

**Table 1: The gradient condition of the mobile phase in Low-resolution mass spectrum analysis**

| Time (min) | A (CH₃CN, 0.1% HCOOH) | B (H₂O, 0.1% HCOOH) |
|---|---|---|
| 0 - 3 | 5 - 100 | 95 - 0 |
| 3 - 6 | 100 | 0 |
| 6 - 6.1 | 100 - 5 | 0 - 95 |
| 6.1 - 8 | 5 | 95 |

The data of the compound purity, reaction product content, or intermediate control product content are obtained by high performance liquid chromatography (HPLC). The high performance liquid chromatography (HPLC) instrument can be an Agilent HPLC system, and the chromatographic column can be Xbridge Phenyl (4.6×150mm, 3.5 µm), ZORBAX Extend-C18 (4.6×150mm, 5 µm), or Waters Xbridge phenyl (4.6×150mm, 3.5 µm); gradient elution is performed using an aqueous phosphoric acid solution (optionally containing potassium dihydrogen phosphate) and acetonitrile as the mobile phase.

The stereoisomers of the present invention are detected using high-performance liquid chromatography. The high-performance liquid chromatography (HPLC) instrument can be an Agilent HPLC system, and the chromatographic column can be selected from OJ-RH (4.6×250mm, 5 µm), Daicel CHIRALPAK IC (4.6×250mm, 5 µm), or Philomans CHIRAL NY (4.6×250mm, 5 µm); gradient elution is performed using trifluoroacetic acid-acetonitrile solution and *n*-hexane as the mobile phase.

### EXAMPLES

The embodiments of the present invention disclose methods for preparing the pyrrole amide compound as shown in Formula (A) and intermediates thereof. Skilled in the art can learn from this invention to properly improve the process parameters to implement the preparation method. Of particular note is that all similar substitutions and modifications to the skilled person is obvious, and they are deemed to be included in the present in invention. Related person can clearly realize and apply the techniques disclosed herein by making some changes, appropriate alterations or combinations to the methods without departing from spirit, principles and scope of the present disclosure.

In order to enable those skilled in the art to further understand the invention, it is detailed below through examples.

### Example

### Example 1 Preparation of

### 2-chloro-1-(2-hydroxyethyl)-4-methyl-5-(2-(trifluoromethyl)phenyl)-1H-pyrrole-3-carboxylic acid (intermediate 1)

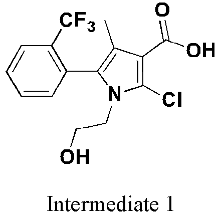

Ethyl 2-chloro-1-(2-hydroxyethyl)-4-methyl-5-(2-(trifluoromethyl)phenyl)-1*H*-pyrrole-3-carboxylate (29.1g, 77.44 mmol) prepared with reference to a well-known method in the art was added to a mixed solvent of methanol (90 mL) and water (30 mL), and then potassium hydroxide (15.34 g, 232.32 mmol, purity 85%) was added and the mixture was heated to reflux for 3 hours. The solvent was evaporated under reduced pressure, water (90 mL) was added to the residue, then the mixture was washed with methyl tert-butyl ether (90 mL), the aqueous phase was adjusted to pH=4 with 2 mol/L hydrochloric acid solution , and then extracted with ethyl acetate (150 mL × 2), the organic phases were combined, and washed with water (100 mL) and saturated brine (100 mL) successively, then dried over anhydrous sodium sulfate and filtered with suction, and the filtrate was concentrated under reduced pressure to obtain a light yellow solid of 26.3 g with a yield of 97.67%.

### Example 2 Preparation of

### (S)-2-chloro-1-(2-hydroxyethyl)-4-methyl-5-(2-(trifluoromethyl)phenyl)-1H-pyrrole-3-carboxylic acid (intermediate 2)

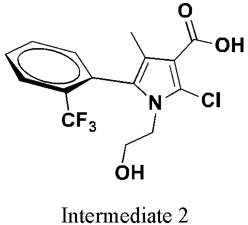

### Method one:

Quinine (7.28 g, 22.43 mmol) was weighed into a reaction flask, *N, N*-dimethylacetamide (6.5 mL), ethyl acetate (65 mL) and water (3.9 mL) were added to the flask and the mixture was heated to 60°C. 2-Chloro-1-(2-hydroxyethyl)-4-methyl-5-(2-(trifluoromethyl)phenyl)-1*H*-pyrrole-3-carboxylic acid (intermediate 1) (13.00 g, 37.39 mmol) was added to a solution of *N, N*-dimethylacetamide (6.5 mL) and ethyl acetate (48 mL), and the mixture was heated and dissolved, and then added dropwise to the aforementioned quinine solution. The heating was turned off, the system was cooled to room temperature, filtered with suction, and the filter cake was rinsed with ethyl acetate (13mL×2) and vacuum-dried. The filter cake was added to the mixed solvent of ethyl acetate (80 mL) and water (60 mL), to which was added 2 mol/L hydrochloric acid solution (18.7 mL) under stirring, the aqueous phase was separated after 10 minutes, the organic phase was washed with water (50 mL) and brine (40 mL) in turn and dried over anhydrous sodium sulfate and filtered with suction, and the filtrate was concentrated under reduced pressure to obtain a light yellow solid of 6.35 g, with an ee value of 96.04%, a purity of 99.65% and a yield of 48.84%.

### Method two

Quinine (83.97 g, 172.55 mmol) was added into N,N-dimethylacetamide (75 mL), ethyl acetate (1050 mL) and water (45 mL), after which the mixture was heated to 65 °C. 2-Chloro-1-(2-hydroxyethyl)-4-methyl-5-(2-(trifluoromethyl)phenyl)-1*H*-pyrrole-3-carboxylic acid (intermediate 1) (150.0 g, 287.59 mmol) was added to a solution of *N,N-*dimethylacetamide (75 mL) and ethyl acetate (255 mL), and the mixture was heated and dissolved, and then added dropwise to the aforementioned quinine solution. After the addition was complete, the mixture was kept at 65 °C with stirring for 1 hour and then cooled to room temperature. The mixture was filtered, and the filter cake was washed with ethyl acetate (50 mL × 2). The filter cake was collected and dried under vacuum at 45 °C for 11 hours to obtain a white solid (131.96 g). 4 M hydrochloric acid solution was added to the obtained white solid until the pH ≤3, and the mixture was stirred at room temperature for 5 hours. The mixture was filtered, and the filter cake was washed with water (500 mL), collected, and vacuum dried at 60 °C for 17 hours to obtain the target product as a white solid (64.83 g, 43.22%).

### Identification of intermediate 2:

MS (ESI, pos. ion) m/z: 314.2 (M+1);
¹H NMR (400 MHz, CDCl3) δ 7.82 (d, *J*= 7.6 Hz, 1H), 7.64 (dt, *J*= 21.3, 7.4 Hz, 2H), 7.43 (d, *J* = 7.3 Hz, 1H), 4.05 (dt, *J* = 10.5, 6.0 Hz, 1H), 3.84 - 3.60 (m, 3H), 2.01 (s, 3H).

### Example 3 Preparation of

### (S)-1-(2-(benzyloxy)ethyl)-2-chloro-4-methyl-5-(2-(trifluoromethyl)phenyl)-1H-pyrrole-3-carboxylic acid benzyl ester (intermediate 3)

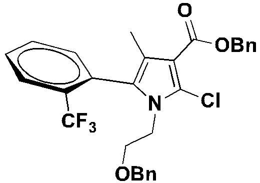

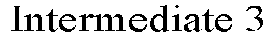

### Method one:

The intermediate 2 (100 g, 287.59 mmol) and benzyl bromide (147.56 g, 862.77 mmol) were dissolved in *N,N*-dimethylacetamide (2 L) under nitrogen protection, and sodium *tert*-amylate (95.02 g, 862.77 mmol) was added. The mixture was then heated to 40 °C and reacted overnight. The reaction solution was poured into water (4000 mL), and then extracted with MTBE (800 mL × 3). The combined organic phases were washed with saturated brine (600 mL × 2), and the solvent was removed by concentration under reduced pressure to give a pale yellow transparent oil (151 g, 99.45%).

### Method two:

The intermediate 2 (8.25 g, 23.73 mmol), benzyl bromide (12.18 g, 71.19 mmol), *N,N*-dimethylacetamide (83 mL) and sodium tert-amylate (7.84 g, 71.19 mmol) were added to the reaction flask. Afterward, the mixture was replaced with nitrogen three times, and then heated to 50 ± 5 °C for 4 hours under nitrogen protection. The reaction mixture was diluted with water (250 mL) and extracted with methyl *tert*-butyl ether (75 mL × 2), and the organic phase was washed with 20% saline solution (200 mL × 2) and concentrated under reduced pressure to obtain the target product as a pale yellow transparent oil (12.52 g, 99.95%).

### Example 4 Preparation of

### (S)-1-(2-(benzyloxy)ethyl)-2-chloro-4-methyl-5-(2-(trifluoromethyl)phenyl)-1H-pyrrole-3-carboxylic acid (intermediate 4)

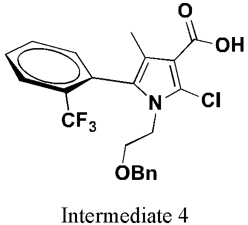

Ethanol (240 mL), potassium hydroxide (35.01 g, 530.36 mmol, 85% purity) and water (60 mL) were added to intermediate 3 (70 g, 132.59 mmol), after which the mixture was heated to 70 °C for 6 hours. The reaction solution was cooled to 45 °C, followed by the addition of water (240 mL) for dilution. Ethanol was removed by rotary evaporation under reduced pressure. Methyl *tert*-butyl ether (400 mL) and heptane (200 mL) were added to a beaker, and the two solvents were mixed thoroughly for subsequent use. Approximately half of the above-mentioned mixed solvent was added to the residue from reduced-pressure rotary evaporation. The mixture was stirred for 5 minutes, allowed to stand, and the upper organic phase was separated and the aqueous phase was retained. The remaining half of the above-mentioned mixed solvent was added to the aqueous phase. The mixture was stirred for 5 minutes, allowed to stand, and then the upper organic phase was separated and the aqueous phase was retained. Dilute hydrochloric acid (2 M, purchased from Chengdu Kelong) was added to the retained aqueous phase to adjust the pH≤3. Ethyl acetate was used for extraction (500 mL). The organic phase was washed with water (200 mL) and saturated brine (200 mL), respectively, and concentrated under reduced pressure to obtain the target product as a brownish-yellow oil (58.03 g, 99.96%). MS (ESI, pos. ion) m/z: 438.1 (M+1).

### Example 5 Preparation of

### (S)-1-(2-(benzyloxy)ethyl)-4-methyl-5-(2-(trifluoromethyl)phenyl)-1H-pyrrole-3-carboxylic acid (intermediate 5)

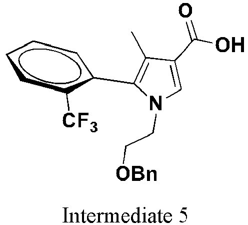

### Instance 1:

Intermediate 4 (12.0 g, 27.41 mmol), sodium formate dihydrate (4.28 g, 41.12 mmol), and palladium-carbon (1.20 g, 1.12 mmol, 10% purity) were dissolved in a mixed solvent of ethanol (120 mL), toluene (60 mL), and water (12 mL). The mixture was heated to 65 °C and reacted for 3.5 hours. The reaction solution was filtered through diatomaceous earth, and the filter cake was washed with ethanol. The filtrate was concentrated under reduced pressure to remove the solvent. The residue was dissolved in ethanol (40 mL), and the pH was adjusted to 2 with 2.5 mol/L hydrochloric acid. The solution was added dropwise to ice water (400 mL), and the mixture was allowed to warm naturally to room temperature and stirred overnight. The mixture was then filtered, and the filter cake was collected and dried under vacuum at 60 °C to obtain the target product as a white solid (9.6 g, 86.83%). HPLC: 96.81%, MS (ESI, pos. ion) m/z: 404.2 (M+1).

¹H NMR (400 MHz, DMSO-*d*₆) δ 11.69 (s, 1H), 7.91 - 7.84 (m, 1H), 7.74 - 7.65 (m, 2H), 7.53 (s, 1H), 7.38 - 7.25 (m, 4H), 7.21 (d, *J* = 6.8 Hz, 2H), 4.46 - 4.35 (m, 2H), 3.92 - 3.81 (m, 1H), 3.60 - 3.44 (m, 3H), 1.88 (s, 3H). Instance 2-5:
Referring to the method in Instance 1, the method conditions for preparing intermediate 5 were tested, wherein the reaction temperature was 60°C, the amount of sodium formate was 1.5 equivalents (i.e., the molar amount of sodium formate was 1.5 times the molar amount of intermediate 4), and the amount of palladium-carbon was 10% (calculated as a mass percentage of raw material intermediate 4); the experimental results are shown in the table below.

**Table A**

| Instance | Feed Amount | Reaction Solvent | Reaction Time | In-Process Control Content |
|---|---|---|---|---|
| Instance 2 | 10g | Toluene | 24h | No detection was performed, more than 50% of the raw material remained. |
| Instance 3 | 10g | Ethanol/water = 10/1 | 3h | 78.22% |
| Instance 3 | 1g | Ethanol/toluene/water = 6/4/1 | 6h | 83.17% |
| Instance 4 | 1g | Ethanol/toluene/water = 8/2/1 | 6h | 81.76% |
| Instance 5 | 1g | Ethanol/toluene/water = 10/5/1 | 4h | 93.88% |

| | | | | |
|---|---|---|---|---|
| Note: In the table above, the ratio of mixed solvents refers to the volume ratio; the in-process control product content refers to the product content in the reaction system after the reaction is completed and before post-treatment, which is the peak area ratio of the product obtained by HPLC detection of samples taken from the reaction system. | | | | |

The compound of Formula (A) can be prepared using intermediate 5 as a raw material according to a similar method disclosed in the prior art.

Reference throughout this specification to "an embodiment," "some embodiments," "one embodiment", "another example," "an example," "a specific examples," or "some examples," means that a particular feature, structure, material, or characteristic described in connection with the embodiment or example is included in at least one embodiment or example of the present disclosure. Thus, the appearances of the phrases such as "in some embodiments," "in one embodiment", "in an embodiment", "in another example, "in an example," "in a specific examples," or "in some examples," in various places throughout this specification are not necessarily referring to the same embodiment or example of the present disclosure.

Although explanatory embodiments have been shown and described, it would be appreciated by those skilled in the art that the above embodiments cannot be construed to limit the present disclosure, and changes, alternatives, and modifications can be made in the embodiments without departing from spirit, principles and scope of the present disclosure.

## Claims

1. A compound of Formula (I) 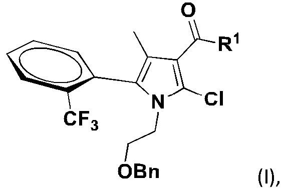 wherein, R¹ is OH, NH₂, C₂₋₄ alkoxy or benzyloxy, each of the C₂₋₄ alkoxy and benzyloxy is optionally substituted with 1, 2, 3 or 4 substituents independently selected from halogen, C₁₋₄ alkyl, C₁₋₄ haloalkyl, C₁₋₄ alkoxy and C₁₋₄ haloalkoxy.

2. A compound having one of the following structures,

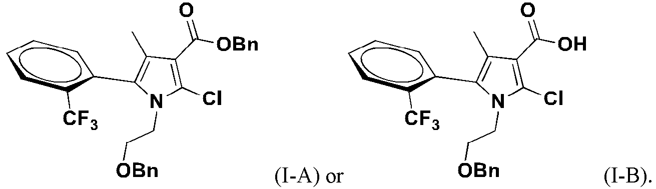

3. A method for preparing the compound of Formula (II), 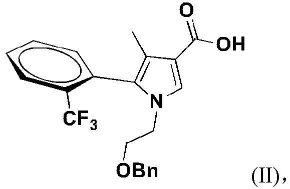 wherein, the method comprises:
Step c) The compound of formula (I-B) is dechlorinated in a solvent under suitable conditions to give the compound of formula (II),

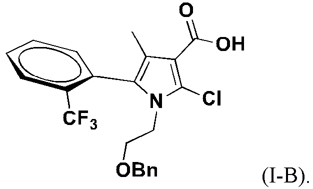

4. The method according to claim 3, wherein the solvent is methanol, ethanol, isopropanol, tert-butanol, or a mixed solvent composed of any of them with other solvent in any combination, wherein the other solvent is tetrahydrofuran, DMF, ethyl acetate, methyl *tert*-butyl ether, ethylene glycol dimethyl ether, ethylene glycol monomethyl ether, toluene, water or any combination thereof;
optionally, the solvent is methanol, ethanol, ethanol/toluene, ethanol/water, ethanol/toluene/water, ethanol/tetrahydrofuran, or ethanol/tetrahydrofuran/water.

5. The method according to claim 3 or 4, wherein the reaction in step c) is carried out in the presence of a transition metal catalyst; optionally, the transition metal catalyst is a palladium-carbon catalyst, a palladium acetate catalyst or a nickel catalyst.

6. The method according to any one of claims 3-5, wherein the reaction in step c) is carried out in the presence of ammonium formate, sodium formate, disodium hydrogen phosphate, acid, triethylamine or hydrogen.

7. The method according to any one of claims 3-6, wherein the reaction in step c) is carried out under heating conditions; optionally, the heating conditions in step c) are heating to 45 °C-75 °C, 55 °C-70 °C or 60 °C-65 °C.

8. The method according to any one of claims 3-7, further comprising a method for preparing the compound of formula (I-B), wherein the method for preparing the compound of formula (I-B) comprises:
Step a) The compound of formula (I-1) reacts with a benzyl protecting group reagent to obtain the compound of formula (I-A); 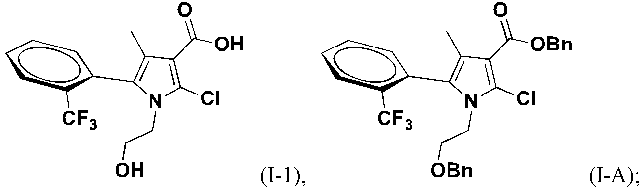
Step b) The compound of formula (I-A) is debenzylated under suitable conditions to obtain the compound of formula (I-B);

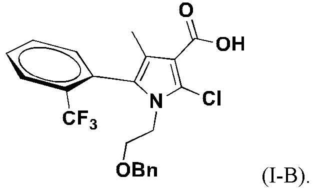

9. The method according to claim 8, wherein the benzyl protecting group reagent in step a) is a halobenzyl group, preferably benzyl bromide;
optionally, the step a) is carried out in the presence of sodium tert-amylate;
optionally, the reaction solvent in step a) is *N,N*-dimethylacetamide;
optionally, the reaction temperature in step a) is 25 °C to 60 °C, or 35 °C to 55 °C, or 45 °C to 55 °C.

10. The method according to claim 8 or 9, wherein the step b) is carried out in the presence of a base, which is sodium carbonate, potassium carbonate, cesium carbonate, potassium phosphate, lithium hydroxide, potassium hydroxide or sodium hydroxide;
optionally, the reaction in step b) is carried out in a solvent, which is acetone, acetonitrile, *N,N*-dimethylformamide, *N,N*-dimethylacetamide, DMSO, methanol, ethanol, THF, methyl *tert*-butyl ether, water or any combination thereof;
optionally, the reaction in step b) is carried out under heating conditions, which mean heating to 60 °C to 80 °C, or 65 °C to 75 °C.

11. The method according to any one of claims 8-10, further comprising a method for preparing the compound of formula (I-1), wherein the method for preparing the compound of formula (I-1) comprises:
1a) The compound of formula (I-0) reacts with an optically active amine to obtain the corresponding salt of the optically active amine of the compound of formula (I-1), and
1b) The salt obtained in step 1a) reacts to give the compound of formula (I-1);

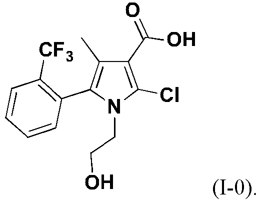

12. The method according to claim 11, wherein the optically active amine is an optically active amine having a quinine skeleton; optionally, the optically active amine having a quinine skeleton is quinine, hydroquinine, quinidine, cinchonine or cinchonidine.

13. The method according to claim 11 or 12, wherein the reaction in step 1b) is a hydrolysis reaction;
optionally, the reaction in step 1b) is carried out under acidic conditions; optionally, the acidic conditions are those for the presence of hydrochloric acid, sulfuric acid, hydrobromic acid or citric acid;
optionally, the reaction solvent in step 1b) is *N,N*-dimethylformamide, *N,N*-dimethylacetamide, acetonitrile, tetrahydrofuran, ethanol, acetone, isopropyl acetate, ethyl acetate, methyl *tert*-butyl ether, water, dimethoxyethane or any combination thereof.

14. The method according to any one of claims 11-13, wherein the reaction solvent in step 1a) is *N,N*-dimethylformamide, *N,N*-dimethylacetamide, acetonitrile, tetrahydrofuran, ethanol, acetone, isopropyl acetate, ethyl acetate, methyl tert-butyl ether, water, dimethoxyethane or any combination thereof;
optionally, the reaction temperature in step 1a) is room temperature to 100 °C; preferably, the reaction temperature in step 1a) is 50 °C to 80 °C or 55 °C to 65 °C.
